(19) Europäisches Patentamt
European Patent Office
Office européen des brevets

(11) **EP 3 758 023 B1**

(12) **EUROPEAN PATENT SPECIFICATION**

(45) Date of publication and mention
of the grant of the patent:
**09.02.2022  Bulletin 2022/06**

(21) Application number: **20183007.2**

(22) Date of filing: **29.06.2020**

(51) International Patent Classification (IPC):
**G16H 50/00** *(2018.01)*     **A41D 1/00** *(2018.01)*
**G06K 9/00** *(2022.01)*

(52) Cooperative Patent Classification (CPC):
**G06V 40/23; G16H 50/00**

(54) **METHOD AND DEVICE FOR ASSESSING PHYSICAL MOVEMENT OF AN OPERATOR DURING A WORK CYCLE EXECUTION ON AN INDUSTRIAL MANUFACTURING WORK STATION**

VERFAHREN UND VORRICHTUNG ZUR BEURTEILUNG DER PHYSIKALISCHEN BEWEGUNG EINES BEDIENERS WÄHREND EINER ARBEITSZYKLUSAUSFÜHRUNG AN EINER INDUSTRIELLEN FERTIGUNGSARBEITSSTATION

PROCÉDÉ ET DISPOSITIF D'ÉVALUATION DE MOUVEMENT PHYSIQUE D'UN OPÉRATEUR LORS DE L'EXÉCUTION D'UN CYCLE DE TRAVAIL SUR UNE STATION DE TRAVAIL DE FABRICATION INDUSTRIELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2019  PT 2019115612**
**30.06.2019  EP 19183514**

(43) Date of publication of application:
**30.12.2020  Bulletin 2020/53**

(73) Proprietor: **Associação Fraunhofer Portugal Research**
**4200-135 Porto (PT)**

(72) Inventors:
• **DOS SANTOS NUNES FOLGADO, Duarte Miguel**
**2670-386 Loures (PT)**

• **DA SILVEIRA BARANDAS, Marília**
**2795-016 Linda A Velha (PT)**
• **FERNANDES CARVALHO, Miguel Ângelo**
**4700-384 Braga (PT)**
• **SILVEIRA GAMBOA, Hugo Filipe**
**1050-034 Lisboa (PT)**

(74) Representative: **Patentree**
**Edificio Net**
**Rua de Salazares, 842**
**4149-002 Porto (PT)**

(56) References cited:
**US-A1- 2007 250 286     US-A1- 2012 323 521**

**Description**

**TECHNICAL FIELD**

The present disclosure relates to the field of monitoring tasks performed by human operators in industrial manufacturing environments. In particular, this disclosure relates to an automated human work analysis method and device in a workplace, by calculating worker performance and ergonomic risk measurements.

**BACKGROUND**

Nowadays, the increasing demand for highly customizable products requires the implementation of flexible, adaptive and preventive manufacturing systems. Over the last few years, there has been a paradigm shift from mass production to mass customization, which has resulted in an increase in the complexity of manufacturing processes and their decision support systems. Access to accurate, real-time information on production control is an important requirement to meet these new challenges. The manufacturing industries rely on movement's libraries that must be followed by the employees who are assigned to conduct those tasks. These movements are well-known and aim to ensure the fulfilment of the productivity goals while minimizing the risk of musculoskeletal injuries associated with repetitive tasks.

For example, document US 2011/0270135 A1 discloses a system and method for neurological testing involves prompting a subject or user to engage in body and head movements, measuring physical response, and then using the measured response to evaluate the neurological condition of the subject. The subject may wear a head-mounted display, for example, to aid in prompting the head and/or body movements. The subject may be prompted to engage in movements simulating a real-world task, such as a sports-specific activity. Results may be compared with those of a baseline test. The evaluation may be used to aid in determining if the user or subject can return to the task, for example to return to activity in a sport during which the user sustained a possible neurological injury.

Document US20060259472A1 also discloses a method and system for analyzing work in a workplace is provided. The method includes providing a worker database; providing a task database for a plurality of tasks, which includes providing one or more task configurations for each of task and including configuration attributes for the task configurations; matching worker attributes with the task configuration attributes; evaluating the match of the worker attributes with the task configuration attributes; and updating the worker database and the task database upon satisfactory matching of worker attributes with the task configuration attributes. The system includes a worker database, wherein the worker database includes one or more worker attributes; a task database for a plurality of tasks; a user interface for accessing the worker database and the task database for entering worker and task information; and an evaluation module that evaluates a match of worker attributes with the task configuration.

Document WO201076313 discloses a device and a method for characterising movements made by an object, in particular a limb of a human being, fitted with at least one accelerometer sensor. In particular, this document uses algorithms for processing the signals from the sensor in order to calculate the length of a step by double integration of the signals according to the direction of the step. This document also mentions that it can also be used for hand movements having variation characteristics close to those of steps.

Documents US 2007/250286 A1 and US 2012/323521 A1 disclose respectively a motion analysis system and gesture recognition system forming part of the closely related prior art.

These facts are disclosed in order to illustrate the technical problem addressed by the present disclosure.

**GENERAL DESCRIPTION**

It is disclosed a method and device for automated work analysis at workstations by detecting user motion to assess performance and risk of injury. The present disclosure comprises multiple wearable motion sensors to measure the user's motion, a centralized hub to collect information from multiple users and a processing node to retrieve performance and ergonomics indexes from collected data. Embodiments of the disclosure compare the movements performed with a given reference to find the degree of similarity and to characterize the movement's correctness in terms of work method and timing. Further embodiments of the disclosure seek to optimize the work performed by subjects, improve performance by looking for errors and automatically present an overview of the performance index of a group of subjects during shifts in work station.

One of the effects of this monitoring system is to retrieve real human motion information of operators executing repetitive tasks on Industrial environments. The advantages lie in the fact a comparison with a given reference worker can be established to retrieve productivity information and ergonomic risk assessment.

One of the aims of the disclosure is to provide the overall cost of an optimized warping path as a dissimilarity score between the captured physical movement in comparison with the previously acquired physical movement template. Deviations between the captured physical movement and the previously recorded reference will increase the dissimilarly score.

A more comprehensive explanation is available with regard to the techniques and algorithms used to characterize human motion in industrial environments according to the present disclosure:

Table I: Enumeration of the features used in the classification task for each type.

| Anomaly | A break in the production process (either planned or unplanned). Unplanned breaks - examples consist of unexpected stop of machines, unexpected lack of raw materials, among others; planned breaks correspond to shift changes periods, operator rest allowances, among others. An anomaly is defined as a break of the cyclic pattern in the time series. |
|---|---|
| Evaluator | An entity present in most modern industrial environments, which integrates the Productions QA team's, namely Work and Method Study department. It aims to ensure that the operator's methods and timings are in accordance with the production requirements. The evaluator must be present in the calibration stage to annotate transitions between different work cycles. |
| Work cycle | An individual repetition of a given task. |
| Work station | The area which surrounds the operator while working. Most of the times the operator interacts with objects, tools and equipment located at the work station to accomplish a given task. |
| Sequence | The time series acquired during the acquisition stage by a given operator working at a given work station. |
| Task | A task is a set of standard movements required to be performed during a given work cycle. |
| Template | Time series acquired in the calibration stage by a reference operator (establishing the standard time and method) or by the same operator of the acquisition stage (establishing the possibility to monitor own methods and timings). |

The disclosed system and method allow evaluation of the degree of movements' correctness performed by a given operator while executing a repetitive task. The evaluation is based on a comparison between a previously recorded template and the movements executed by the subject under evaluation. The outputs are scores that quantify the degree of similarity between the template and work cycles under evaluation.

The comparison aims to evaluate work cycles in two domains:

Spatial: ensures the method used for work cycle execution is similar with the template.
Temporal: ensures that the timing in which the method is executed is similar with the template.

The following pertains to the assumptions and mathematical foundations for the two measurements.

The term spatial is adopted with regard to the method executed to complete a given work cycle. The following assumptions are taken into account: (1) similar movement execution will result in similar accelerations, therefore similar work cycles have similar acceleration profiles; (2) similar movement execution will result in similar rotations, therefore similar work cycles will have similar gyroscope profiles; (3) similar movement execution will result in similar sensor spatial location, therefore similar work cycles will have similar magnetometer profiles.

It is worth mentioning a more detailed explanation of point (3). Earth's geomagnetic field has intrinsic magnetic variability and is affected by ferromagnetic interferences. In industrial environments, the geomagnetic field is therefore distorted (e.g. sewing machines, structural surfaces on work stations, displacement of tools, etc.). Therefore, the spatial surroundings of a work station have increased geomagnetic variability. When a magnetometer sensor travels through the work station space, it registers a "magnetic signature" of the movement. Therefore, similar movements will have similar geomagnetic field values, since they follow the same spatial trajectory.

After the segmentation process is finished, all the retrieved work cycles are compared against the template. The spatial similarity is calculated using Equation 1:

$$DTW(X,Y) = \frac{1}{K}\sqrt{\sum_{k=1}^{K} w_k^*}$$

During the execution of a work cycle, there exists temporal variation associated with each of the movements that compose the cycle. The term temporal similarity refers to the evaluation of the timing in which the movements are executed. Even on circumstances in which the temporal duration between the template and the work cycle being compared is similar, variability may still be present during its length - an operator might execute a work cycle excessively fast on the beginning and excessively slowly on the end.

The present disclosure comprises time series measurement to express the similarity in temporal domain between two time series which is designated as Time Alignment Measurement (TAM). The measurement quantifies the degree of temporal distortion between two time series.

In an embodiment, two sequences $X$ of length $N \in \mathbb{N}$ and $Y$ of length MEN can be considered. During the complete length of each sequence, the signals may be considered to exhibit phase and out of phase behaviours. In case the signals are out of phase, one sequence can be considered to be in advance in relation to the other. This characteristic is reciprocal as if $X$ is in advance in relation to $Y$, $Y$ can be considered to be delayed in relation to $X$.

The TAM measures the percentage of length at which a given signal is in delay, advance and phase in relation to the template. This distance penalizes signals where advance or delay is present and benefits series that are in phase between each other. As the distance increases, the dissimilarity between both signals also increases. In case two time series are identical in time, the value of TAM is 0; the maximum value is 3 and represents the theoretical maximum warping between two time series, as described in Equation 2.

$$\Gamma \in \{\mathbb{R}_0^+ | \Gamma \in [0:3]\}$$

After the segmentation process is finished, the retrieved work cycles are compared against the template. The temporal similarity is calculated using TAM.

An aspect of the present disclosure relates to a computer-implemented method for assessing physical movement of a task executed by an operator during a work cycle execution at an industrial manufacturing work station in comparison with a previously acquired physical movement template and previously identified representative instants in said previously acquired physical movement template referred to as template synchronization instants which include start and end instants of the movement template, comprising:

capturing a data sequence comprising a plurality of time series of movement data from a tri-axial output inertial sensor being worn by the operator, the data sequence comprising a time series for each of the sensor outputs along periodical instants for a predetermined period of time;

calculating candidate representative instants in said captured sequence referred to as candidate synchronization instants;

aligning said plurality of time series with a previously acquired data sequence comprising a plurality of time series of template movement data, by:

computing a cost matrix comprising multidimensional Euclidean distance between each pair of instants for the captured sequence and the template sequence;

finding an optimized warping path between the captured sequence and the template sequence;

wherein a warping path is defined using said cost matrix as a continuous path that matches each instant of the captured sequence with an instant of the template sequence in said cost matrix;

wherein the finding of an optimized warping path between the captured sequence and the template sequence comprises:

generating sub-warping paths using said cost matrix between the candidate synchronization instants;

selecting the sub-warping paths that minimizes a predefined cost metric and, when merged, provide a full warping path between the captured sequence and the template sequence;

providing the overall cost of the optimized warping path as a dissimilarity score between the captured physical movement in comparison with the previously acquired physical movement template.

One of the aims of the disclosure is to provide the overall cost of an optimized warping path as a dissimilarity score between the captured physical movement in comparison with the previously acquired physical movement template. Deviations between the captured physical movement and the previously recorded reference will increase the dissimilarly score.

In an embodiment, the method of the present disclosure comprises the step of determining an anomaly in the physical movement of a task executed by an operator during a work cycle execution, if the dissimilarity score is above a predetermined threshold.

In an embodiment, selecting the sub-warping paths that minimize cost and when merged provide a full warping path between the captured sequence and the template sequence, comprises the following steps:

for all sub-warping paths that share the same end point, choosing the sub-warping path that has the least cost metric and discarding the rest;
for each set of sub-warping paths that are overlaid in time, select the sub-warping path that has the least cost metric.

In an embodiment, selecting the sub-warping paths that minimize cost and when merged provide a full warping path between the captured sequence and the template sequence, further comprises the following steps, for repetitive movements in the captured sequence wherein multiple optimized warping paths between the captured sequence and the template sequence will be found:

calculating the time difference between end and start instants of consecutive optimized warping paths;
if the calculated time difference is lower or equal than a predetermined threshold, adjusting the captured sequence such that the end and start instants are the same;
if the calculated time difference is higher than the predetermined threshold, classifying the time between the end and start instants as an anomaly.

In an embodiment, the predefined sub-warping path cost metric is calculated by adding, in particular by weighted adding, one or more of the following:

calculating the cost of the sub-warping path using said cost matrix between the candidate synchronization instants;
calculating the cost of the sub-warping path using the sub accumulated cost matrix between start and end points of the sub-warping path;
length of the sub-warping path;
costs of the respective candidate synchronization instants.

It is noted that the tri-axial inertial sensor comprises a tri-axial accelerometer, a tri-axial gyroscope and a tri-axial magnetometer.

In an embodiment, the method of the present disclosure may comprise applying a weighted cost function to apply differentiated weights to the tri-axial inertial sensor outputs.

In an embodiment, the method of the present disclosure may comprise applying a weighted cost function to apply differentiated weights to the first order derivative of each sequence.

In an embodiment, the method of the present disclosure may comprise applying a low-pass filter to the tri-axial inertial sensor output, in particular a low-pass filter having a 1Hz cut-off frequency.

In an embodiment, the method of the present disclosure may comprise calculating synchronization instants from the template sequence by carrying out the following steps:

obtaining a self-similarity matrix comprised of the multidimensional Euclidean distance between each instant for the captured sequence and the rest of the sequence;
for each line of the self-similarity matrix, computing a histogram;
obtaining a self-similarity metric by calculating the cumulative sum of a predetermined initial percentage of total number of bins per line, in particular calculating the cumulative sum of the initial 20% of total number of bins per line;
finding the local minima of the obtained self-similarity metric;
selecting the synchronization instants from the found local minima.

Another aspect of the present disclosure, relates to a device for assessing physical movement of a task executed by an operator during a work cycle execution on an industrial manufacturing work station in comparison with a previously acquired physical movement template and previously identified representative instants in said previously acquired physical movement template referred as template synchronization instants which include start and end instants of the movement template, the device comprising a tri-axial output inertial sensor for wearing by the operator and an electronic data processor arranged for carrying out:

capturing a data sequence comprising a plurality of time series of movement data from the tri-axial output inertial sensor comprising a time series for each of the sensor outputs along periodical instants for a predetermined period of time;

calculating candidate representative instants in said captured sequence referred as candidate synchronization instants;

aligning said plurality of time series with a previously acquired data sequence comprising a plurality of time series of template movement data, by:

computing a cost matrix comprised of the multidimensional Euclidean distance between each pair of instants for the captured sequence and the template sequence;

finding an optimized warping path between the captured sequence and the template sequence;

wherein a warping path is defined using said cost matrix as a continuous path that matches each instant of the captured sequence with an instant of the

template sequence in said cost matrix;

wherein the finding an optimized warping path between the captured sequence and the template sequence comprises:

generating sub-warping paths using said cost matrix between the candidate synchronization instants;

selecting the sub-warping paths that minimizes a predefined cost metric and, when merged, provide a full warping path between the captured sequence and the template sequence;

assessing the physical movement is achieved by calculating the overall cost of the optimized warping path as a dissimilarity score between the captured physical movement in comparison with the previously acquired physical movement template.

In an embodiment, the electronic data processor is further arranged for selecting the sub-warping paths that minimize cost and when merged provide a full warping path between the captured sequence and the template sequence, by carrying out the following steps:

for all sub-warping paths that share the same end point, choosing the sub-warping path that has the least cost metric and discarding the rest;

for each set of sub-warping paths that are overlaid in time, select the sub-warping path that has the least cost metric.

In an embodiment, the electronic data processor is further arranged for selecting the sub-warping paths that minimize cost and when merged provide a full warping path between the captured sequence and the template sequence, carrying out the following steps, for repetitive movements in the captured sequence, wherein multiple optimized warping paths between the captured sequence and the template sequence will be found:

calculating the time difference between end and start instants of consecutive optimized warping paths;

if the calculated time difference is lower or equal than a predetermined threshold, adjusting the captured sequence such that the end and start instants are the same;

if the calculated time difference is higher than the predetermined threshold, classifying the time between the end and start instants as an anomaly.

In an embodiment, the electronic data processor is further arranged for calculating the predefined sub-warping path cost metric by adding, in particular by weighted adding, one or more of the following:

calculating the cost of the sub-warping path using said cost matrix between the candidate synchronization instants;

calculating the cost of the sub-warping path using the sub accumulated cost matrix between start and end points of the sub-warping path;

length of the sub-warping path;

costs of the respective candidate synchronization instants.

In an embodiment, the electronic data processor is further arranged for applying a weighted cost function to apply differentiated weights to the tri-axial inertial sensor outputs, in particular applying a weighted cost function to also apply differentiated weights to the first order derivative of each sequence.

Another aspect of the present disclosure relates to a method of the disclosure which comprises calculating synchronization instants from the template sequence by carrying out the following steps:

obtaining a self-similarity matrix comprised of the multidimensional Euclidean distance between each instant for the captured sequence and the rest of the sequence;

for each line of the self-similarity matrix, computing a histogram;
obtaining a self-similarity metric by calculating the cumulative sum of a predetermined initial percentage of total number of bins per line, in particular calculating the cumulative sum of the initial 20% of total number of bins per line;
finding the local minima of the obtained self-similarity metric;
selecting the synchronization instants from the found local minima.

Non-transitory storage media including program instructions for implementing a method for assessing physical movement of a task executed by an operator during a work cycle execution on an industrial manufacturing work station, the program instructions including instructions executable by a data processor to carry out cording to the method of the present disclosure.

It is disclosed a method of providing a model of standard work cycle execution for human operators comprising:

a. An a priori model is constructed for each task by registering the human movements of a given operator. This model is used as a baseline for comparison and is designated as "reference operator".
b. The model is constructed after several work cycle iterations to build a template, which is the most representative of adequate human performance execution of a given task on a given work station.
c. The template model can be modified to integrate temporal allowances for work cycle execution.

It is also disclosed a system of providing a quantitative analysis of an operator's performance while conducting a task on Industrial manufacturing work stations:

a. Obtaining a set of operator's real-time measurements to track tri-dimensional Human motion.
b. A quantitative analysis is performed based in the comparison of the operator's performance against the model described.
c. The comparison is able to perform a quantitative analysis based on distance measurements on temporal and spatial domains.
d. The total number of repetitions is also automatically calculated.
e. A break of repetitive pattern is designated as "anomaly" and is automatically identified.

The disclosure is also characterized by a combination of sensor fusion techniques and opportunistic information regarding magnetic field to improve analysis performance:

a. There is provided an apparatus suitable to estimate Human movement based on magnetic field static interferences at the user surrounding space.
b. The user surrounding space comprises the close and finite environment which is delimited by the freedom of movements that the user can actually perform.
c. Earth's magnetic field possess natural and local interferences arising from structural and architectural properties. Those interference can be detected using a magnetometer sensor and they therefore model the magnetic field around the finite user surrounding volume. Without artificial or prior modification on the magnetic environment is hereby possible to extract Human motion information. The information retrieved from the magnetometer sensor is used to establish an estimation of the path of the sensor and therefore the anatomical segment's location where the sensor is placed.

The disclosure includes a complete system design comprising one or more software elements and hardware operable, when required in monitoring environment.

a. capture inertial sensor data of the subject undertaking a given manufacturing repetitive task
b. Transmit motion data to a distributed processing architecture
c. A centralized server is used to collect and store processed data
d. User interface for presenting processed data including, but not limited to: total number of repetitions, similarity score in comparison with a given reference worker of the method used, similarity score in comparison

## BRIEF DESCRIPTION OF THE DRAWINGS

The following figures provide preferred embodiments for illustrating the disclosure and should not be seen as limiting the scope of invention.

**Figure 1:** Schematic representation of an embodiment of modules: acquisition, processing and visualization.

**Figure 2:** Schematic representation of an embodiment wearable unit. The device consists of an inertial sensor unit and an induction coil for wireless charging. The x axis is referenced in 1, y axis is referenced in 3 and z axis is referenced in 2.

**Figure 3:** Schematic representation of an embodiment of real data on Industrial environments. The triaxial accelerometer is represented in the first set of three lines, the triaxial gyroscope is represented in the second set of three lines and the triaxial magnetometer is represented in the third set of three lines. The black vertical lines correspond to transitions between work cycles. Data was filtered with a zero phase 2nd order low pass Butterworth filter with cut-off frequency of 0.1Hz.

**Figure 4:** Schematic representation of accumulated cost matrix between two time series using the Euclidean distance as local cost measure. The resulted optimal warped path follows the low-cost regions (represented in white) and avoids high cost regions (represented in dark).

**Figure 5:** Schematic representation of self-similarity cost matrix of a given template. Horizontal bars highlight instants that will be further discussed in the present disclosure.

**Figure 6:** Schematic representation of an embodiment of: (a) histogram of line 105 and (b) histogram of line 318.

**Figure 7:** Schematic representation of an embodiment of synchronization instant calculation based on time series self-similarity. Low values indicate instants which are unique during the complete time series length.

**Figure 8**: Schematic representation of an embodiment of cost matrix between a template and a sequence on Industrial environment. It is possible to identify a repetitive pattern corresponding to the several iteration of work cycles performed. The black circles consist of candidates similar with the associated synchronization instants of the reference. Sub warping paths are calculated between black circles (represented in dark and white). The dark sub warping path was selected, and the white candidates were not selected.

**Figure 9**: Schematic representation of an embodiment of cost matrix of between a template and a sequence on Industrial environment. Warping paths are identified by lines of different colour: dark warping paths were previously selected by our selection metric. Note the darker lines corresponding to warping paths that are overlaid in time.

**Figure 10:** Schematic representation an embodiment of cost matrix of between a template and a sequence on Industrial environment. The final warping paths are represented in dark and correspond to the work cycles which were identified. It is possible to conclude that each subsequence has different optimal alignment paths, thus resulting in different temporal variability. An anomaly is present between instants around 2600 and 2800, possibly a pause. There is no optimal alignment path present during the anomaly.

## DETAILED DESCRIPTION

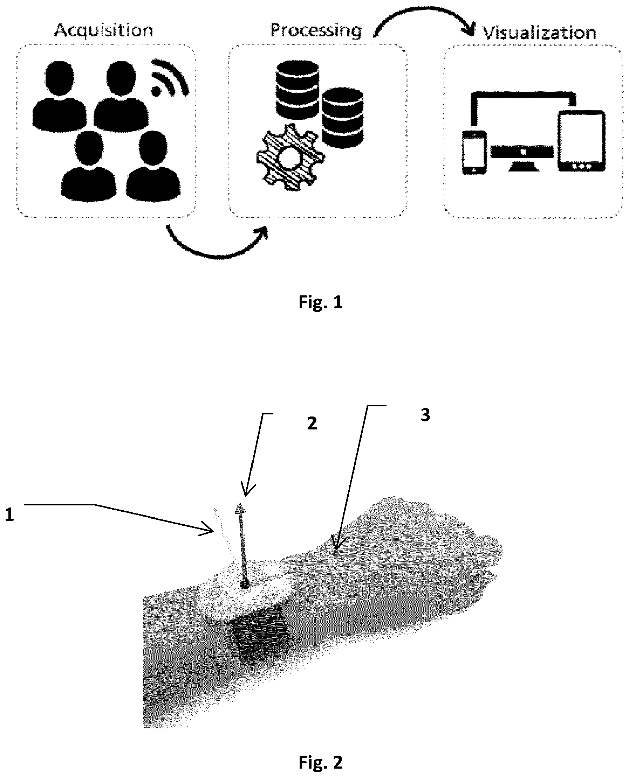

**Figure 1** shows a schematic representation of an embodiment of the system architecture where: **1** represents acquisition, which is responsible to collect and transmit data to a centralized server; **2** represents processing, which integrates a layer of intelligence to extract knowledge from the acquired data, and **3** represents visualization, composed by a user interface to control and visualize the results of the acquisitions process.

The pipeline to set-up the system preferably comprises a calibration stage and an acquisition stage.

The following pertains to the calibration stage: the calibration stage aims to create the template that will be used to identify and characterize each work cycle performed by the operator. During this stage, a calibration acquisition is performed by an operator working on a given work station. Since in this initial stage there is no a priori knowledge regarding the method used to accomplish the task, it requires the presence of an evaluator to annotate the transitions between consecutive work cycles.

The template may be saved either by (1) a highly qualified reference operator or (2) the same operator being evaluated:

1. Reference operator: a highly qualified worker establishes the observed time and method to perform a specific task. The observed time is used to calculate the standard time, which is the time required by an average skilled operator, working at a normal pace, to perform a specified task using a prescribed method. It includes appropriate allowances to allow the person to recover from fatigue and, where necessary, an additional allowance to cover contingent elements which may occur but have not been observed. This template is used to quantify the degree of similarity with the standard time and standard method required to accomplish a given task.

2. Personal template: a template is created based on the movements performed by the operator himself. Therefore, it might not strictly coincide with the standard movements required for a given task. This template may be used to monitor the individual worker own rhythm and own method during the day.

Both methodologies aim to measure the correct movement execution, more precisely, if the method being used is in accordance with the standard method and if there are no movements being performed that may prejudice ergonomics.

In order to establish a representative template, the operator needs to perform a series of repetitions (approx. 10 work cycles) in the presence of an evaluator. The calibration stage protocol is required for each work station and it is described as follows:

1. The evaluator describes to the operator the standard method to execute a given task;

2. The operator starts performing consecutive work cycles;

3. At the instants which correspond to transitions between work cycles (i.e. end of current work cycle and start of consecutive work cycle), the evaluator annotates this event by "tapping"2 in a user interface;

4. After a representative number of repetitions the protocol terminates and data is ready to be processed to construct the motion template. The algorithms used to build the template are presented.

The following pertains to the acquisition stage: After establishing a template for a given work station, it is possible to start the acquisition stage. Industrial facilities have integrated information systems to manage shift planning, therefore, it is possible to establish associations between the registered operators and the work stations they are assigned to.

Thus, it is not required further interaction to setup the acquisition stage. The wearable preferably has an integrated "wake on motion" feature, which transmits data only in case there is movement detected. This feature aims to improve the usability of the solution, since it significantly reduces the interaction required to setup the system without compromising the battery autonomy of the device - in fact, there is no interaction required by the operator.

In case the wearable detects movement, it sends the data to the processing module. The processing is performed in near real time: a buffer of inertial data is processed periodically by the processing server. The period can be adjusted in accordance with user requirements; however, it should be higher than the standard time to accomplish a given task (in order to ensure that the segmentation algorithm identifies at least one occurrence of the template in the sequence buffer).

**Figure 2** shows a schematic representation of an embodiment of the wearable and sensor coordinate system. The wearable device is composed by a 9 DOF inertial unit: includes a tri-axial accelerometer, tri-axial gyroscope and tri-axial magnetometer. The sensor is able to stream data continually at 25Hz during a complete working day. The sensor is located on the most informative anatomical segment for the operation. Typically, on instrumental tasks the sensor is located on the dominant wrist of the operator.

The data is sent preferably using Bluetooth Low Energy to nearby devices defined as edge nodes. These nodes are responsible to collect data up to a maximum of, for example, 7 wearables. After data is collected by the edge nodes, it is sent to a centralized server for processing and storage. Therefore, for large industrial environments, several edge nodes are required (1 node for each 7 wearables).

A detailed explanation of the algorithms used to automatically identify work cycles based on template search on a sequence and characterize each of the instances.

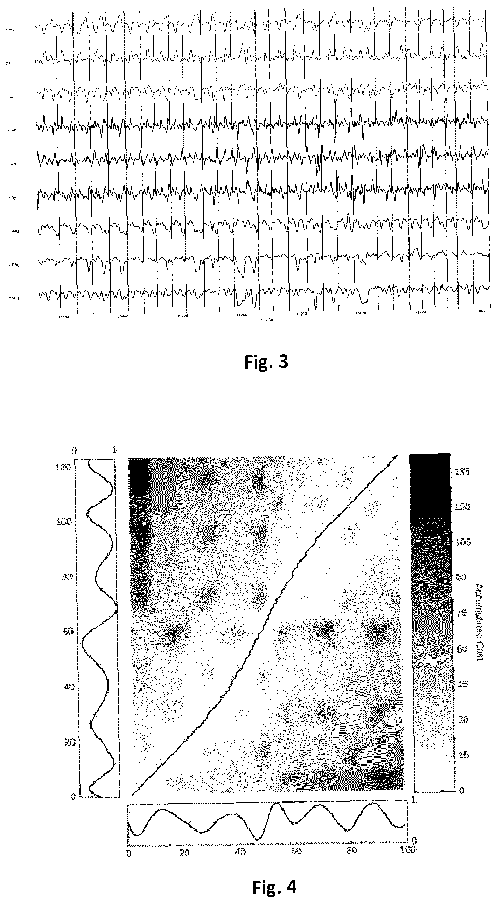

Let $S:=(s1,s2,...,sN)$ represent a multidimensional time series of length $N$. One can define the data vector, $X$, as a 9-dimensional vector composed by temporal synchronized data of the 9DOF inertial sensor unit, comprised by several instances of S. An example of real data is present on **Figure 3.**

$$X := \begin{bmatrix} ACC_0^x & ACC_1^x & ... & ACC_N^x \\ ACC_0^y & ACC_1^y & ... & ACC_N^y \\ ACC_0^z & ACC_1^z & ... & ACC_N^z \\ GYR_0^x & GYR_1^x & ... & GYR_N^x \\ GYR_0^y & GYR_1^y & ... & GYR_N^y \\ GYR_0^z & GYR_1^z & ... & GYR_N^z \\ MAG_0^x & MAG_1^x & ... & MAG_N^x \\ MAG_0^y & MAG_1^y & ... & MAG_N^y \\ MAG_0^z & MAG_1^z & ... & MAG_N^z \end{bmatrix} \quad (eq. \ 1)$$

In order to express similarity between time series a distance measure must be used.

The disclosed Dynamic Time Warping (DTW) algorithm allows two time-dependent sequences that are similar, but locally out of phase, to align in time. One of its main objectives consist of identifying an optimal alignment between sequences by warping the time axis iteratively.

In order to align two time series $X := (x1, x2, ..., xN)$ and $Y := (y1, y2, ..., yN)$ of length N and M respectively, a N-by-M cost matrix is computed. Each (nth, mth) element of the cost matrix, $C \in RN \times M$ corresponds to the distance between each pair of elements of the sequences X and Y. The Euclidean distance is usually employed as a distance function to define the cost matrix element as:

$$c(xn, ym) = (xn - ym)^2 \text{ (eq. 2)}$$

In the present specific case, since the two time series are multidimensional (composed by data streams of 3 sensors in 3 axis), a multidimensional distance is used. Furthermore, in order to improve algorithm's robustness, we modified the cost function to take into account the first derivative as described in Equation 3.

$$c(x_n, y_m) = \alpha \times \sqrt{\sum_{i=0}^{9} \Upsilon \left( \frac{x_n}{\bar{x}} - \frac{y_m}{\bar{y}} \right)^2} + (1 - \alpha) \times \sqrt{\sum_{i=0}^{9} \Upsilon \left( \frac{dx_n}{dX} - \frac{dy_m}{dY} \right)^2} \quad \text{(eq. 3)}$$

Where $\alpha \in \mathbb{R}0+ \cap [0:1]$ is a constant that defines the weighting between the cost in amplitude and first order derivative, dX and dY are the first order derivatives of X and Y, respectively and $\bar{X}, \bar{Y}, d\bar{\bar{X}}$ and $d\bar{\bar{Y}}$ are the L2 norms of each vector. Y is the weight vector.

Regarding the weight vector, it is worth to present a more detailed description. Different types of sensors and respective orientation might lead to unequal contributions regarding their importance for the overall cost. For a given work station, one sensor might be considered as more important in comparison with another if it is more informative. Therefore, Y, summarizes the relative weight of each sensor and orientation and it is composed by a 9 column matrix as follows:

$$\Upsilon = \begin{bmatrix} 1 \\ 1 \\ 1 \\ 1 \\ 1 \\ 1 \\ 1 \\ 1 \\ 1 \end{bmatrix} \quad \text{(eq. 4)}$$

In the case of Equation 4, all sensors and orientations contribute equally to the overall cost. Finding the most suitable set of weights for each work station is non-trivial.

The goal of DTW is to find the optimal warping alignment path between X and Y having minimum overall cost. A warping path, W, is a set of matrix elements that define the relationship between X and Y. The kth element of W is defined as $wk = (i,j)k, wk \in \mathbb{R}2$.

$$W = (w_1, w_2, \ldots, w_k) \qquad max(N, M) \leq K \leq N + M - 1 \quad \text{(eq. 5)}$$

The optimal warping path is the path that has the minimum total cost among all possible warping paths. One could test every incumbent warping path and determine the minimum cost candidate, but such method will lead to an exponential computational complexity in the lengths of N and M. Using dynamic programming, an accumulated cost matrix, D, is computed in order to find the path that minimizes the warping cost in an O(N,M) complexity.

Each accumulated cost matrix element is defined as the local cost measure in the current cell plus the minimum of the local cost measures in the adjacent cells. **Figure 4** represents an example with two synthetic time series.

The DTW distance should be normalized In order to compensate the effect of different optimal warping path lengths according to Equation 6.

$$DTW(X,Y) = \frac{1}{K}\sqrt{\sum_{k=1}^{K} w_k^*} \quad (\text{Eq. 6})$$

The template creation is a system's calibration step required to setup the disclosed method and device. It aims to create a representative human motion template represented by a multidimensional vector with similar shape to Equation 1. Let $R \in M \times N$ define such vector, where $M$ represents the number of inertial axis and $N$ the length of the sequence.

After completing the calibration stage protocol, it is possible to have an annotated dataset consisting of the several work cycle repetitions and the knowledge with regards to instants in which a transition occurs. This fact allows to have the a priori knowledge required to construct the template.

Below, the method to build a reference is described according to an aspect of the disclosure.

The following pertains to pre-processing. The retrieved signals are preferably collected at 25Hz and are preferably pre-processed using a zero-phase 2nd order Butterworth digital filter with a cut-off frequency of 1 Hz. This process aims to improve the signal to noise ratio and clean the input signal in order to access the Human motion lowest frequencies.

Afterwards, data is statistically standardized. For each line of matrix $R$ the following standardization is applied:

$$for\ i\ in\ 1\ ...\ M:$$

$$R_i = \frac{R_{i,N} - \mu_{R_{i,N}}}{\sigma_{R_{i,N}}}$$

The aforementioned steps aim to prepare the signal in order to improve the automatic segmentation algorithm.

The following pertains to representative work cycle selection. In case several work cycle repetitions are performed one can create the template using two methods:

- Average all the signal after previous signal alignment in temporal domain; or
- Select the most representative work cycle. The most representative work cycle consists of the repetition in which the initial and final instants are most aligned with the annotated instants by the Evaluator.

The following pertains to synchronization instants segmentation. After the representative work cycle is created, a last step is performed to calculate synchronization instants. One can define a synchronization instant in a time series, the instants that correspond to the most unique points of the time series (i.e. which are most unique in comparison with the rest of the signal).

Synchronization instants are, therefore, instants in the time series that are unique and will be used afterwards in the automatic segmentation algorithm to help identifying the occurrences of the template on a longer sequence.

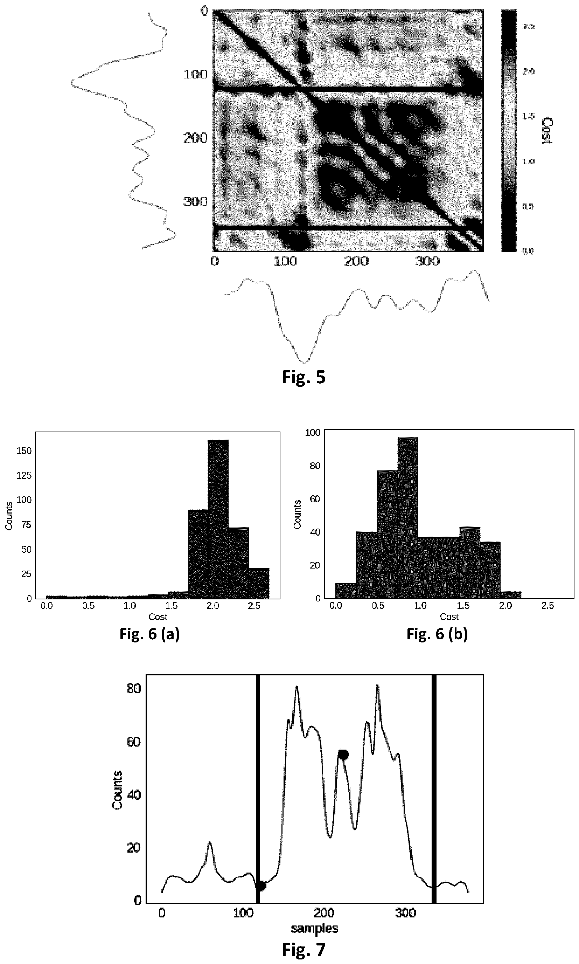

The synchronization instants may also be calculated with an algorithm known in the state of the art or by heuristic or by inspection (maximum and minimum values, peaks, valleys, etc). Figure 5 represents the self-similarity matrix of a given template. Each cell corresponds to the distance of each point to the rest of the time series.

For each line of the self-similarity matrix, we compute a Histogram with 10 bins. Figure 6(a) and Figure 6(b) summarize two examples. The histogram of line 105 is significantly more shifted to the right in comparison with line 318. This fact arises since line 105 corresponds to an instant of higher uniqueness, as the cost has a number of high values in comparison with low values.

Thereafter, we retrieve the cumulative sum of the initial 20% of total number of bins per line, being able to calculate the degree of uniqueness of each sample.

**Figure** 7 illustrates the self-similarity metric. Low values indicate instants which are unique during the complete time series length. This fact is the case of sample 105, which was already discussed on Figure 7. In case of sample 318, there is a high value of the self-similarity metric and, therefore, we can consider that this sample is not much unique, as expected by the behaviour of its histogram represented on **Figure 7.**

Firstly, the calibration stage protocol must be preferably performed, in which an operator executes a series of repetitions whereas an evaluator annotates the instants that corresponds to transitions between work cycles.

Secondly, the cycle which is most representative is calculated preferably based on two methods - global averaging or maximum alignment.

Lastly, a self-similarity matrix is constructed to calculate synchronization instants, which correspond to instants of maximum uniqueness in comparison with the rest of the sequence. Those instants will be used in the template-based segmentation.

After the template creation it is possible to proceed with the segmentation phase, which consists of detecting several work cycles repetitions automatically by identifying the instants in time where the transitions occur. Below, the method to detect each work cycle is described according to an aspect of the disclosure.

The following pertains to pre-processing. Since the sequence signals are directly compared with the template signals, the pre-processing specifications applied to the sequence signals are preferably equal to the ones previously described.

The following pertains to sub warping paths selection. The segmentation algorithm starts with cost matrix calculation between the template and the sequence being analysed. The a priori knowledge of the template instants that correspond to the most unique points in time, defined by synchronization instants, are used to help identifying the corresponding occurrences on the sequence. The identification of the most probable synchronization instants of the entire sequence are defined by the local minima of each synchronization line in the cost matrix, i.e. sequence instants which are more similar with the template synchronization instants.

For each candidate of the sequence synchronization instants, a sub warping path is calculated. One can define a sub warping path in a cost matrix, the warping path calculated between two consecutive points (i.e. two candidates of the sequence synchronization instants). The sub warping paths are computed assuming that the duration of each work cycle segment is limited to a lower and upper time tolerance. The tolerance values are calculated based on the expected duration of each segment, which is extracted by the a priori knowledge of the template.

Finally, each candidate of the sequence's synchronization instants will compromise a varied number of possible sub warping paths. We thereafter retrieve metrics for each sub warping path and select the one that minimizes our selection metric. The selection metric depends on the following measurements:

- Sub warping path cost using the cost matrix between the template and the sequence;
- Sub warping path cost using the sub accumulated cost matrix (i.e. accumulated cost matrix calculated between the start and end points of the sub warping path);
- Sub warping path length;
- Cost value of both candidates of the sequence synchronization instants.

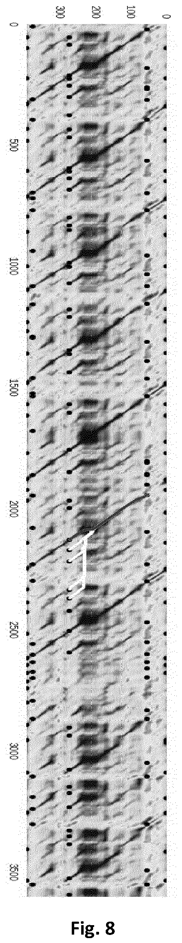

**Figure 8** illustrates the selection process of the sub warping paths where the cost matrix between the template and the sequence is presented. The black dots represent the candidates of sequence synchronization instants and the lines, darker and lighter, represent the sub warping paths calculated from one sequence synchronization instant. The darker line also shows the selected sub warping path.

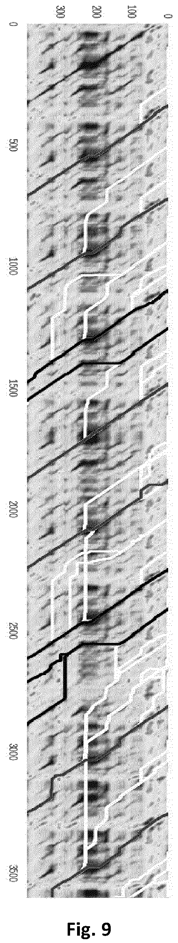

The following pertains to full warping paths selection. After all sub warping paths are selected, a merging process between segments that have extremes in common is applied. Since there are several combinations of possible warping paths (all lines with different colours presented in **Figure 9**), the first step is to reduce the number of possible combinations by grouping warping paths with the same end point and selecting the one that minimizes our selection metric previously described above (dark lines in **Figure 9**).

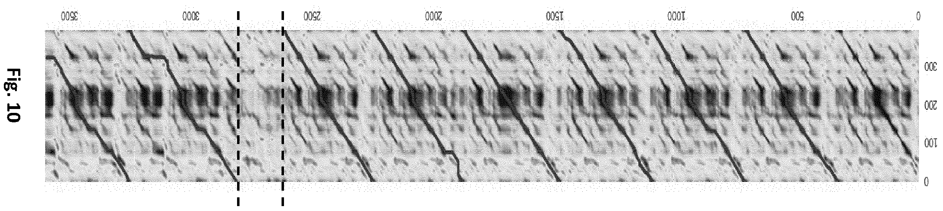

The next step is to detect the warping paths overlaid in time, i.e. warping paths that start before the end of the previous warping path. These warping paths overlaid in time are represented in black in **Figure 9**. Similarly, with the previous steps, the selecting process is done using our selection metric and the full warping paths are obtained and shown in **Figure 10**. The full warping paths can be costed using the cost matrix from beginning to end of the path by summing the total cost of the path over the cost matrix.

The following pertains to final subsequence path calculation. The segmentation algorithm ends with a fine refinement of the start and end points of the obtained warping paths. Since the temporal information is used to produce the output scores of a given work cycle and the movements are performed in a repetitive way, every time instant of the acquisition needs to belong to a specific work cycle or to be classified as an anomaly event. In this sense, a comparison between the time instants of the end and start points of consecutives warping paths is performed. If the time difference found is below a predefined threshold, an adjustment is applied ensuring that a new work cycle starts immediately after the end of the previously one. Otherwise, if the time difference between two consecutive work cycles is greater than the predefined threshold, the instants in time between these two consecutive warping paths are classified as an anomaly event. In **Figure 10,** the time instants between the black dash lines is classified as an anomaly event.

It is thus disclosed an algorithm capable to automatically detect all the work cycles performed on a given sequence. One of the main objectives is to find all the occurrences of the template in the sequence. Even on the circumstances that a break of the repetitive pattern is present, the algorithm was able to correctly identify the work cycles. Each work cycle is then preferably characterized by similarity metrics as known in the art based on the data provided as disclosed above or from page 26.

The term "comprising" whenever used in this document is intended to indicate the presence of stated features, integers, steps, components, but not to preclude the presence or addition of one or more other features, integers, steps, components or groups thereof.

Flow diagrams of particular embodiments of the presently disclosed methods are depicted in figures. The flow diagrams do not depict any particular means, rather the flow diagrams illustrate the functional information one of ordinary skill in the art requires to perform said methods required in accordance with the present disclosure.

It will be appreciated by those of ordinary skill in the art that unless otherwise indicated herein, the particular sequence of steps described is illustrative only and can be varied without departing from the disclosure. Thus, unless otherwise stated the steps described are so unordered meaning that, when possible, the steps can be performed in any convenient or desirable order.

It is to be appreciated that certain embodiments of the disclosure as described herein may be incorporated as code (e.g., a software algorithm or program) residing in firmware and/or on computer useable medium having control logic for enabling execution on a computer system having a computer processor, such as any of the servers described herein. Such a computer system typically includes memory storage configured to provide output from execution of the code which configures a processor in accordance with the execution. The code can be arranged as firmware or software, and can be organized as a set of modules, including the various modules and algorithms described herein, such as discrete code modules, function calls, procedure calls or objects in an object-oriented programming environment. If implemented using modules, the code can comprise a single module or a plurality of modules that operate in cooperation with one another to configure the machine in which it is executed to perform the associated functions, as described herein.

The disclosure should not be seen in any way restricted to the embodiments described and a person with ordinary skill in the art will foresee many possibilities to modifications thereof. The above described embodiments are combinable. The following claims further set out particular embodiments of the disclosure.

**Claims**

1.  A computer-implemented method for assessing physical movement of a task executed by an operator during a work cycle execution on an industrial manufacturing work station in comparison with a previously acquired physical movement template and previously identified representative instants in said previously acquired physical movement template referred as template synchronization instants which include start and end instants of the movement template, comprising:

    capturing a data sequence comprising a plurality of time series of movement data from a tri-axial output inertial sensor being worn by the operator, the data sequence comprising a time series for each of the sensor outputs along periodical instants for a predetermined period of time;
    calculating candidate representative instants in said captured sequence referred as candidate synchronization instants;
    aligning said plurality of time series with a previously acquired data sequence comprising a plurality of time series of template movement data, by:

      computing a cost matrix comprised of the multidimensional Euclidean distance between each pair of instants for the captured sequence and the template sequence;
      finding an optimized warping path between the captured sequence and the template sequence; wherein a warping path is defined using said cost matrix as a continuous path that matches each instant of the captured sequence with an instant of the template sequence in said cost matrix; wherein the finding of the optimized warping path between the captured sequence and the template sequence comprises:

        generating sub-warping paths using said cost matrix between the candidate synchronization instants;
        selecting the sub-warping paths that minimize a predefined cost metric and, when merged, provide a full warping path between the captured sequence and the template sequence;

    providing the overall cost of the optimized warping path as a dissimilarity score between the captured physical movement in comparison with the previously acquired physical movement template.

2.  Method according to the previous claim comprising the step of determining an anomaly in the physical movement of a task executed by an operator during a work cycle execution, if the dissimilarity score is above a predetermined threshold.

3.  Method according to any of the previous claims wherein selecting the sub-warping paths that minimize cost and when merged provide a full warping path between the captured sequence and the template sequence, comprises the following steps:

    for all sub-warping paths that share the same end point, choosing the sub-warping path that has the least cost metric and discarding the rest;
for each set of sub-warping paths that are overlaid in time, select the sub-warping path that has the least cost metric.

4. Method according to any of the previous claims wherein selecting the sub-warping paths that minimize cost and when merged provide a full warping path between the captured sequence and the template sequence, further comprises the following steps, for repetitive movements in the captured sequence wherein multiple optimized warping paths between the captured sequence and the template sequence will be found:

calculating the time difference between end and start instants of consecutive optimized warping paths;
if the calculated time difference is lower or equal than a predetermined threshold, adjusting the captured sequence such that the end and start instants are the same;
if the calculated time difference is higher than the predetermined threshold, classifying the time between the end and start instants as an anomaly.

5. Method according to any of the previous claims wherein the predefined sub-warping path cost metric is calculated by adding, in particular by weighted adding, one or more of the following:

calculating the cost of the sub-warping path using said cost matrix between the candidate synchronization instants;
calculating the cost of the sub-warping path using the sub accumulated cost matrix between start and end points of the sub-warping path;
length of the sub-warping path;
costs of the respective candidate synchronization instants.

6. Method according to any of the previous claims which comprises applying a weighted cost function to apply differentiated weights to the first order derivative of each sequence.

7. Method according to any of the previous claims which comprises applying a low-pass filter to the tri-axial inertial sensor output, in particular a low-pass filter having a 1Hz cut-off frequency.

8. Method according to any of the previous claims which comprises calculating synchronization instants from the template sequence by carrying out the following steps:

obtaining a self-similarity matrix comprised of the multidimensional Euclidean distance between each instant for the captured sequence and the rest of the sequence;
for each line of the self-similarity matrix, computing a histogram;
obtaining a self-similarity metric by calculating the cumulative sum of a predetermined initial percentage of total number of bins per line, in particular calculating the cumulative sum of the initial 20% of total number of bins per line;
finding the local minima of the obtained self-similarity metric;
selecting the synchronization instants from the found local minima.

9. Device for assessing physical movement of a task executed by an operator during a work cycle execution on an industrial manufacturing work station in comparison with a previously acquired physical movement template and previously identified representative instants in said previously acquired physical movement template referred as template synchronization instants which include start and end instants of the movement template, the device comprising a tri-axial output inertial sensor for wearing by the operator and an electronic data processor arranged for carrying out:

capturing a data sequence comprising a plurality of time series of movement data from the tri-axial output inertial sensor comprising a time series for each of the sensor outputs along periodical instants for a predetermined period of time;
calculating candidate representative instants in said captured sequence referred as candidate synchronization instants;
aligning said plurality of time series with a previously acquired data sequence comprising a plurality of time series of template movement data, by:

computing a cost matrix comprised of the multidimensional Euclidean distance between each pair of instants for the captured sequence and the template sequence;

finding an optimized warping path between the captured sequence and the template sequence;

wherein a warping path is defined using said cost matrix as a continuous path that matches each instant of the captured sequence with an instant of the

template sequence in said cost matrix;

wherein the finding an optimized warping path between the captured sequence and the template sequence comprises:

generating sub-warping paths using said cost matrix between the candidate synchronization instants;

selecting the sub-warping paths that minimizes a predefined cost metric and, when merged, provide a full warping path between the captured sequence and the template sequence;

providing the overall cost of the optimized warping path as a dissimilarity score between the captured physical movement in comparison with the previously acquired physical movement template.

10. Device according to the previous claim wherein the electronic data processor is further arranged for selecting the sub-warping paths that minimize cost and when merged provide a full warping path between the captured sequence and the template sequence, by carrying out the following steps:

for all sub-warping paths that share the same end point, choosing the sub-warping path that has the least cost metric and discarding the rest;

for each set of sub-warping paths that are overlaid in time, select the sub-warping path that has the least cost metric.

11. Device according to any of the claims 9-10 wherein the electronic data processor is further arranged for selecting the sub-warping paths that minimize cost and when merged provide a full warping path between the captured sequence and the template sequence, carrying out the following steps, for repetitive movements in the captured sequence, wherein multiple optimized warping paths between the captured sequence and the template sequence will be found:

calculating the time difference between end and start instants of consecutive optimized warping paths;

if the calculated time difference is lower or equal than a predetermined threshold, adjusting the captured sequence such that the end and start instants are the same;

if the calculated time difference is higher than the predetermined threshold, classifying the time between the end and start instants as an anomaly.

12. Device according to any of the claims 9-11 wherein the electronic data processor is further arranged for calculating the predefined sub-warping path cost metric by adding, in particular by weighted adding, one or more of the following:

calculating the cost of the sub-warping path using said cost matrix between the candidate synchronization instants;

calculating the cost of the sub-warping path using the sub accumulated cost matrix between start and end points of the sub-warping path;

length of the sub-warping path;

costs of the respective candidate synchronization instants.

13. Device according to any of the claims 9-12 wherein the electronic data processor is further arranged for applying a weighted cost function to apply differentiated weights to the tri-axial inertial sensor outputs, in particular applying a weighted cost function to also apply differentiated weights to the first order derivative of each sequence.

14. Device according to any of the claims 9-13 wherein the electronic data processor is further arranged for calculating synchronization instants from the template sequence by carrying out the following steps:

obtaining a self-similarity matrix comprised of the multidimensional Euclidean distance between each instant for the captured sequence and the rest of the sequence;

for each line of the self-similarity matrix, computing a histogram;

obtaining a self-similarity metric by calculating the cumulative sum of a predetermined initial percentage of total number of bins per line, in particular calculating the cumulative sum of the initial 20% of total number of bins per line;

finding the local minima of the obtained self-similarity metric;

selecting the synchronization instants from the found local minima.

15. Non-transitory storage media including program instructions for implementing a method for assessing physical movement of a task executed by an operator during a work cycle execution on an industrial manufacturing work station, the program instructions including instructions executable by a data processor to carry out the method of any of the claims 1-8.

**Patentansprüche**

1. Ein computerimplementiertes Verfahren zur Beurteilung der physischen Bewegung einer Aufgabe, die von einem Bediener während einer Arbeitszyklusausführung an einer industriellen Fertigungsarbeitsstation ausgeführt wird, im Vergleich zu einem zuvor erfassten physischen Bewegungsmuster und zuvor identifizierten repräsentativen Punkten bei dem genannten zuvor erfassten physischen Bewegungsmuster, die als Mustersynchronisationspunkte bezeichnet werden und Anfangs- und Endpunkte des Bewegungsmusters beinhalten, umfassend:

Erfassen einer Datensequenz, umfassend eine Vielzahl von Zeitreihen von Bewegungsdaten eines vom Bediener getragenen dreiachsigen Trägheitssensors, wobei die Datensequenz eine Zeitreihe für jeden der Sensoren entlang regelmäßiger Punkte für einen vorgegebenen Zeitraum umfasst;

Berechnen repräsentativer Kandidatenpunkte in der genannten erfassten Sequenz, die als Kandidaten-Synchronisationspunkte bezeichnet werden;

Abgleichen der genannten Vielzahl an Zeitreihen mit einer zuvor erfassten Datensequenz, umfassend eine Vielzahl von Zeitreihen von Musterbewegungsdaten, durch:

Berechnen einer Kostenmatrix, bestehend aus dem mehrdimensionalen euklidischen Abstand zwischen jedem Zeitpaar der erfassten Sequenz und der Mustersequenz;

Ermitteln eines optimierten Warping-Pfads zwischen der erfassten Sequenz und der Mustersequenz; wobei ein Warping-Pfad unter Verwendung der genannten Kostenmatrix als ein kontinuierlicher Pfad definiert ist, bei dem jeder Punkt der erfassten Sequenz mit einem Punkt der Mustersequenz in der genannten Kostenmatrix übereinstimmt; wobei die Ermittlung des optimierten Warping-Pfads zwischen der erfassten Sequenz und der Mustersequenz umfasst:

Erzeugen von Sub-Warping-Pfaden unter Verwendung der genannten Kostenmatrix zwischen den Kandidaten-Synchronisationspunkten;

Auswählen des Sub-Warping-Pfads, der eine vorgegebene Kostenmetrik minimiert und, wenn zusammengeführt, einen vollständigen Warping-Pfad zwischen der erfassten Sequenz und der Mustersequenz liefert;

Bereitstellen der Gesamtkosten des optimierten Warping-Pfads als Wert der Abweichung zwischen der erfassten physischen Bewegung und dem zuvor erfassten physischen Bewegungsmuster.

2. Verfahren nach dem vorangehenden Anspruch, umfassend den Schritt der Bestimmung einer Anomalie in der physischen Bewegung einer Aufgabe, die von einem Bediener während einer Arbeitszyklusausführung ausgeführt wird, wenn der Wert der Abweichung einen vorgegebenen Schwellenwert überschreitet.

3. Verfahren nach einem der vorangehenden Ansprüche, wobei die Auswahl der kostenminimierenden Sub-Warping-Pfade, wenn diese zusammengeführt werden, einen vollständigen Warping-Pfad zwischen der erfassten Sequenz und der Mustersequenz liefert, umfassend die folgenden Schritte:

Auswählen des Sub-Warping-Pfads mit der kleinsten Kostenmetrik für alle Sub-Warping-Pfade mit demselben Endpunkt und Verwerfen des Rests;

Auswählen des Sub-Warping-Pfads mit der kleinsten Kostenmetrik für jeden Satz zeitlich überlagerter Sub-Warping-Pfade.

4. Verfahren nach einem der vorangehenden Ansprüche, wobei die Auswahl der kostenmindernden Sub-Warping-Pfade, wenn diese zusammengeführt werden, einen vollständigen Warping-Pfade zwischen der erfassten Sequenz und der Mustersequenz liefert, ferner folgende Schritte für sich wiederholende Bewegungen in der erfassten Sequenz umfassend, wobei mehrere optimierte Warping-Pfade zwischen der erfassten Sequenz und der Mustersequenz gefunden werden:

Berechnen der Zeitverschiebung zwischen End- und Anfangspunkt aufeinanderfolgender optimierter Warping-Pfade;
Anpassen der erfassten Sequenz, wenn die berechnete Zeitverschiebung kleiner oder gleich einem vorgegebenen Schwellenwert ist, sodass der End- und der Anfangspunkt gleich sind;
wenn die berechnete Zeitverschiebung größer ist als der vorgegebene Schwellenwert, wird die Zeit zwischen dem End- und dem Anfangspunkt als Anomalie eingestuft.

5. Verfahren nach einem der vorangehenden Ansprüche, wobei die vorgegebene Kostenmetrik des Sub-Warping-Pfads durch Addieren, insbesondere durch gewichtetes Addieren einer oder mehrerer der folgenden Punkte berechnet wird:

Berechnen der Kosten des Sub-Warping-Pfads unter Verwendung der genannten Kostenmatrix zwischen den Kandidaten-Synchronisationspunkten;
Berechnen der Kosten des Sub-Warping-Pfads unter Verwendung der akkumulierten Kostenmatrix zwischen den Anfangs- und Endpunkten des Sub-Warping-Pfads;
Länge des Sub-Warping-Pfads;
Kosten der entsprechenden Kandidaten-Synchronisationspunkte.

6. Verfahren nach einem der vorangehenden Ansprüche, umfassend die Anwendung einer gewichteten Kostenfunktion, um differenzierte Gewichtungen auf die Ableitung erster Ordnung jeder Sequenz anzuwenden.

7. Verfahren nach einem der vorangehenden Ansprüche, umfassend die Anwendung eines Tiefpassfilters auf den Ausgangswert des dreiachsigen Trägheitssensors, insbesondere eines Tiefpassfilters mit einer Grenzfrequenz von 1 Hz.

8. Verfahren nach einem der vorangehenden Ansprüche, umfassend die Berechnung von Synchronisationspunkten aus der Mustersequenz durch Ausführen folgender Schritte:

Erhalten einer Selbstähnlichkeitsmatrix, bestehend aus dem mehrdimensionalen euklidischen Abstand zwischen jedem Zeitpaar der erfassten Sequenz und dem Rest der Sequenz;
für jede Zeile der Selbstähnlichkeitsmatrix unter Berechnung eines Histogramms;
Erhalten einer Selbstähnlichkeitsmetrik durch Berechnen der kumulativen Summe eines vorgegebenen anfänglichen Prozentsatzes der Gesamtzahl der Bins je Zeile, insbesondere durch Berechnen der kumulativen Summe der anfänglichen 20 % der Gesamtzahl der Bins je Zeile;
Ermitteln der lokalen Minima der erhaltenen Selbstähnlichkeitsmetrik;
Auswählen der Synchronisierungspunkte aus den ermittelten lokalen Minima.

9. Vorrichtung zur Beurteilung der physischen Bewegung einer Aufgabe, die von einem Bediener während einer Arbeitszyklusausführung an einer industriellen Fertigungsarbeitsstation ausgeführt wird, im Vergleich zu einem zuvor erfassten physischen Bewegungsmuster und zuvor identifizierten repräsentativen Punkten bei dem genannten zuvor erfassten physischen Bewegungsmuster, die als Mustersynchronisationspunkte bezeichnet werden und Anfangs- und Endpunkte des Bewegungsmusters beinhalten, wobei die Vorrichtung einen vom Bediener getragenen dreiachsigen Trägheitssensor sowie einen elektronischen Datenprozessor umfasst, der so angeordnet ist, dass er:

eine Datensequenz erfasst, umfassend eine Vielzahl von Zeitreihen von Bewegungsdaten des dreiachsigen Trägheitssensor, wobei die Datensequenz eine Zeitreihe für jeden der Sensoren entlang regelmäßiger Punkte für eine vorgegebene Zeitspanne umfasst;
repräsentative Kandidatenpunkte in der genannten erfassten Sequenz berechnet, die als Kandidaten-Synchronisationspunkte bezeichnet werden;
die genannte Vielzahl an Zeitreihen mit einer zuvor erfassten Datensequenz, umfassend eine Vielzahl von Zeitreihen von Musterbewegungsdaten, abgleicht durch:

Berechnen einer Kostenmatrix, bestehend aus dem mehrdimensionalen euklidischen Abstand zwischen jedem Zeitpaar der erfassten Sequenz und der Mustersequenz;

Ermitteln des optimierten Warping-Pfads zwischen der erfassten Sequenz und der Mustersequenz;

wobei ein Warping-Pfad unter Verwendung der Kostenmatrix als ein kontinuierlicher Verlauf definiert ist, bei dem jeder Punkt der erfassten Sequenz mit einem Punkt der Mustersequenz in der genannten Kostenmatrix übereinstimmt;

wobei die Ermittlung eines optimierten Warping-Pfads zwischen der erfassten Sequenz und der Mustersequenz umfasst:

Erzeugen von Sub-Warping-Pfaden unter Verwendung der genannten Kostenmatrix zwischen den Kandidaten-Synchronisationspunkten;

Auswählen des Sub-Warping-Pfads, der eine vorgegebene Kostenmetrik minimiert und, wenn zusammengeführt, einen vollständigen Warping-Pfad zwischen der erfassten Sequenz und der Mustersequenz liefert;

Bereitstellen der Gesamtkosten des optimierten Warping-Pfads als Wert der Abweichung zwischen der erfassten physischen Bewegung und dem zuvor erfassten physischen Bewegungsmuster.

10. Vorrichtung nach einem der vorangehenden Ansprüche, wobei der elektronische Datenprozessor ferner so angeordnet ist, dass er die kostenmindernden Sub-Warping-Pfade auswählt und, wenn zusammengeführt, einen vollständigen Warping-Pfad zwischen der erfassten Sequenz und der Mustersequenz liefert, durch Ausführen der folgenden Schritte:

Auswählen des Sub-Warping-Pfads mit der kleinsten Kostenmetrik für alle Sub-Warping-Pfade mit demselben Endpunkt und Verwerfen des Rests;

Auswählen des Sub-Warping-Pfads mit der kleinsten Kostenmetrik für jeden Satz zeitlich überlagerter Sub-Warping-Pfade.

11. Vorrichtung nach einem der vorangehenden Ansprüche 9-10, wobei der elektronische Datenprozessor ferner so angeordnet ist, dass er die kostenmindernden Sub-Warping-Pfade auswählt und, wenn zusammengeführt, einen vollständigen Warping-Pfad zwischen der erfassten Sequenz und der Mustersequenz liefert, ferner folgende Schritte für sich wiederholende Bewegungen in der erfassten Sequenz umfasst, wobei mehrere optimierte Warping-Pfade zwischen der erfassten Sequenz und der Mustersequenz gefunden werden:

Berechnen der Zeitverschiebung zwischen End- und Anfangspunkt aufeinanderfolgender optimierter Warping-Pfade;

Anpassen der erfassten Sequenz, wenn die berechnete Zeitverschiebung kleiner oder gleich einem vorgegebenen Schwellenwert ist, sodass der End- und der Anfangspunkt gleich sind;

wenn die berechnete Zeitverschiebung größer ist als der vorgegebene Schwellenwert, wird die Zeit zwischen dem End- und dem Anfangspunkt als Anomalie eingestuft.

12. Vorrichtung nach einem der Ansprüche 9-11, wobei der elektronische Datenprozessor ferner so angeordnet ist, dass er die vorgegebene Kostenmetrik des Sub-Warping-Pfads durch Addieren, insbesondere durch gewichtetes Addieren einer oder mehrerer der folgenden Punkte berechnet:

Berechnen der Kosten des Sub-Warping-Pfads unter Verwendung der genannten Kostenmatrix zwischen den Kandidaten-Synchronisationspunkten;

Berechnen der Kosten des Sub-Warping-Pfads unter Verwendung der akkumulierten Kostenmatrix zwischen den Anfangs- und Endpunkten des Sub-Warping-Pfads;

Länge des Sub-Warping-Pfads;

Kosten der entsprechenden Kandidaten-Synchronisationspunkte.

13. Vorrichtung nach einem der Ansprüche 9-12, wobei der elektronische Datenprozessor ferner so angeordnet ist, dass er eine gewichtete Kostenfunktion anwendet, um differenzierte Gewichtungen auf die dreiachsigen Trägheitssensoren anzuwenden, insbesondere eine gewichtete Kostenfunktion, um auch differenzierte Gewichtungen auf die Ableitung erster Ordnung jeder Sequenz anzuwenden.

14. Vorrichtung nach einem der Ansprüche 9-13, wobei der elektronische Datenprozessor ferner so angeordnet ist, Synchronisationspunkte der Mustersequenz zu berechnen, indem er folgende Schritte ausführt:

Erhalten einer Selbstähnlichkeitsmatrix, bestehend aus dem mehrdimensionalen euklidischen Abstand zwischen jedem Zeitpaar der erfassten Sequenz und dem Rest der Sequenz;
für jede Zeile der Selbstähnlichkeitsmatrix unter Berechnung eines Histogramms;
Erhalten einer Selbstähnlichkeitsmetrik durch Berechnen der kumulativen Summe eines vorgegebenen anfänglichen Prozentsatzes der Gesamtzahl der Bins je Zeile, insbesondere durch Berechnen der kumulativen Summe der anfänglichen 20 % der Gesamtzahl der Bins je Zeile;
Ermitteln der lokalen Minima der erhaltenen Selbstähnlichkeitsmetrik;
Auswählen der Synchronisierungspunkte aus den ermittelten lokalen Minima.

15. Nichtflüchtiges Speichermedium, einschließlich Programmanweisungen zur Implementierung eines Verfahrens zur Beurteilung der physischen Bewegung einer Aufgabe, die von einem Bediener während einer Arbeitszyklusausführung an einer industriellen Fertigungsarbeitsstation ausgeführt wird, wobei die Programmanweisungen Anweisungen enthalten, die von einem Datenprozessor ausgeführt werden können, um das Verfahren nach einem der Ansprüche 1-8 auszuführen.


**Revendications**

1. Un procédé mis en oeuvre par ordinateur pour l'évaluation de mouvement physique d'une tâche exécutée par un opérateur lors de l'exécution d'un cycle de travail sur une station de travail de fabrication industrielle en comparaison avec un modèle de mouvement physique préalablement acquis et des instants représentatifs préalablement identifiés dans ledit modèle de mouvement physique préalablement acquis référés en tant qu'instants de synchronisation de modèle qui incluent des instants de début et de fin du modèle de mouvement, comprenant :

capturer une séquence de données comprenant une pluralité de séries chronologiques de données de mouvement à partir d'un capteur inertiel de sortie triaxiale porté par l'opérateur, la séquence de données comprenant une série chronologique pour chacune des sorties de capteur au long des instants périodiques pour une période de temps prédéterminée ;
calculer les instants représentatifs candidats dans ladite séquence capturée référés en tant qu'instants de synchronisation candidats ;
aligner ladite pluralité de séries chronologiques avec une séquence de données préalablement acquise comprenant une pluralité de séries chronologiques de données de mouvement modèle, en :

calculant une matrice de coût constituée de la distance Euclidienne multidimensionnelle entre chaque paire d'instants pour la séquence capturée et la séquence modèle ;
trouvant un chemin de déformation optimisé entre la séquence capturée et la séquence modèle; dans lequel un chemin de déformation est défini à l'aide de ladite matrice de coût en tant que chemin continu qui associe chaque instant de la séquence capturée à un instant de la séquence modèle dans ladite matrice de coût;
dans lequel la découverte du chemin de déformation optimisé entre la séquence capturée et la séquence modèle comprend :

générer des chemins de sous-déformation à l'aide de ladite matrice de coût entre les instants de synchronisation candidats ;
sélectionner les chemins de sous-déformation qui minimisent une donnée de coût prédéfinie et qui, lorsqu'ils sont fusionnés, fournissent un chemin de déformation complet entre la séquence capturée et la séquence modèle ;

fournir le coût total du chemin de déformation optimisé en tant que résultat de dissimilitude entre le mouvement physique capturé en comparaison avec le modèle de mouvement physique préalablement acquis.

2. Procédé selon la revendication précédente, comprenant l'étape de détermination d'une anomalie dans le mouvement physique d'une tâche exécutée par un opérateur durant une exécution de cycle de travail, si le résultat de dissimilitude est supérieur à une limite prédéterminée.

**3.** Procédé selon l'une quelconque des revendications précédentes, dans lequel la sélection des chemins de sous-déformation qui minimisent le coût et qui, lorsqu'ils sont fusionnés, fournissent un chemin de déformation total entre la séquence capturée et la séquence modèle, comprend les étapes suivantes:

pour tous les chemins de sous-déformation qui partagent le même point final, choisir le chemin de sous-déformation qui représente la donnée de coût moindre et écarter le reste ;
pour chaque ensemble de chemins de sous-déformation qui sont superposés dans le temps, sélectionner le chemin de sous-déformation qui a la donnée de coût moindre.

**4.** Procédé selon l'une quelconque des revendications précédentes, dans lequel la sélection des chemins de sous-déformation qui minimisent le coût et qui, lorsqu'ils sont fusionnés, fournissent un chemin de déformation total entre la séquence capturée et la séquence modèle, comprend également les étapes suivantes, pour les mouvements répétitifs dans la séquence capturée dans laquelle multiples chemins de déformation optimisés entre la séquence capturée et la séquence modèle seront trouvés :

calculer la différence de temps entre les instants de fin et de début de chemins de déformation optimisée consécutifs ;
si la différence de temps calculée est inférieure ou égale à une limite prédéterminée,
ajuster la séquence capturée telle que les instants de fin et de début soient les mêmes ;
si la différence de temps calculée est supérieure à la limite prédéterminée, classifier le temps entre les instants de fin et de début comme une anomalie.

**5.** Procédé selon l'une quelconque des revendications précédentes, dans lequel la donnée de coût du chemin de sous-déformation prédéfini est calculée en additionnant, en particulier en additionnant de façon pondérée, un ou plusieurs des éléments suivants :

calculer le coût du chemin de sous-déformation à l'aide de ladite matrice de coût entre les instants de synchronisation candidats ;
calculer le coût du chemin de sous-déformation à l'aide de la matrice de coût sous-accumulée entre les points de début et de fin du chemin de sous-déformation ;
longueur du chemin de sous-déformation ;
coûts des instants de synchronisation candidats respectifs.

**6.** Procédé selon l'une quelconque des revendications précédentes qui comprend l'application d'une fonction de coût pondéré pour appliquer des pondérations différentiées à la première dérivée d'ordre de chaque séquence.

**7.** Procédé selon l'une quelconque des revendications précédentes qui comprend l'application d'un filtre passe-bas à la sortie de capteur inertiel triaxial, en particulier un filtre passe-bas ayant une fréquence de coupure de 1Hz.

**8.** Procédé selon l'une quelconque des revendications précédentes qui comprend le calcul des instants de synchronisation à partir de la séquence modèle en exécutant les étapes suivantes :

obtenir une matrice d'auto-similarité constituée de la distance Euclidienne multidimensionnelle entre chaque paire d'instants pour la séquence capturée et le reste de la séquence ;
pour chaque ligne de la matrice d'auto-similarité, informatiser un histogramme ;
obtenir une donnée d'auto-similarité en calculant la somme cumulative d'un pourcentage initial prédéterminé d'un nombre total de bacs par ligne, en particulier en calculant la somme cumulative des 20% initiaux du nombre total de bacs par ligne;
trouver le minimum local de la donnée d'auto-similarité obtenue ;
sélectionner les instants de synchronisation à partir du minimum local trouvé.

**9.** Dispositif pour l'évaluation de mouvement physique d'une tâche exécutée par un opérateur lors de l'exécution d'un cycle de travail sur une station de travail de fabrication industrielle en comparaison avec un modèle de mouvement physique préalablement acquis et des instants représentatifs préalablement identifiés dans ledit modèle de mouvement physique préalablement acquis référés en tant qu'instants de synchronisation de modèle qui incluent des instants de début et de fin du modèle de mouvement, le dispositif comprenant un capteur inertiel de sortie triaxial devant être porté par l'opérateur et un traiteur de données électronique arrangé pour exécuter les éléments suivants :

capturer une séquence de données comprenant une pluralité de séries chronologiques de données de mouvement à partir du capteur inertiel de sortie triaxial comprenant une série chronologique pour chacune des sorties de capteur au long d'instants périodiques pour une période de temps prédéterminée ;

calculer des instants représentatifs candidats dans ladite séquence capturée référés en tant qu'instants de synchronisation candidats ;

aligner ladite pluralité de séries chronologiques avec une séquence de données préalablement acquise comprenant une pluralité de séries chronologiques de données de mouvement modèle, en :

calculant une matrice de coût constituée de la distance Euclidienne multidimensionnelle entre chaque paire d'instants pour la séquence capturée et la séquence modèle ;

trouvant un chemin de déformation optimisé entre la séquence capturée et la séquence modèle;

dans lequel un chemin de déformation est défini à l'aide de ladite matrice de coût en tant que chemin continu qui associe chaque instant de la séquence capturée à un

instant de la séquence modèle dans ladite matrice de coût ;

dans lequel le fait de trouver un chemin de déformation optimisé entre la séquence capturée et la séquence modèle comprend:

générer des chemins de sous-déformation à l'aide de ladite matrice de coût entre les instants de synchronisation candidats ;

sélectionner les chemins de sous-déformation qui minimisent une mesure de coût prédéfinie et qui, lorsqu'ils sont fusionnés, fournissent un chemin de déformation complet entre la séquence capturée et la séquence modèle ;

fournir le coût total du chemin de déformation optimisé en tant que résultat de dissimilitude entre le mouvement physique capturé en comparaison avec le modèle de mouvement physique préalablement acquis.

**10.** Dispositif selon la revendication précédente, dans lequel le traiteur de données électronique est également arrangé pour sélectionner les chemins de sous-déformation qui minimisent les coûts et qui, lorsqu'ils sont fusionnés, fournissent un chemin de déformation complet entre la séquence capturée et la séquence modèle, en exécutant les étapes suivantes :

pour tous les chemins de sous-déformation qui partagent le même point de fin, choisir le chemin de sous-déformation qui a la donnée de coût moindre et écarter le reste ;

pour chaque ensemble de chemins de sous-déformation qui sont superposés dans le temps, sélectionner le chemin de sous-déformation qui a la donnée de coût moindre.

**11.** Dispositif selon l'une quelconque des revendications 9-10, dans lequel le traiteur de données électronique est également arrangé pour sélectionner les chemins de sous-déformation qui minimisent le coût et qui, lorsqu'ils sont fusionnés, fournissent un chemin de déformation total entre la séquence capturée et la séquence modèle, exécutant les étapes suivantes, pour les mouvements répétitifs dans la séquence capturée, dans laquelle multiples chemins de déformation optimisés entre la séquence capturée et la séquence modèle seront trouvés :

calculer la différence de temps entre les instants de fin et de début des chemins de déformation optimisés consécutifs ;

si la différence de temps calculée est inférieure ou égale à une limite prédéterminée, ajuster la séquence capturée telle que les instants de fin et de début soient les mêmes ;

si la différence de temps calculée est supérieure à la limite prédéterminée, classifier le temps entre les instants de fin et de début comme une anomalie.

**12.** Dispositif selon l'une quelconque des revendications 9-11, dans lequel le traiteur de données électronique est également arrangé pour calculer la donnée de coût du chemin de sous-déformation en additionnant, en particulier en additionnant de façon pondérée, un ou plusieurs des éléments suivants :

calculer le coût du chemin de sous-déformation à l'aide de ladite matrice de coût entre les instants de synchronisation candidats ;

calculer le coût du chemin de sous-déformation à l'aide de la matrice de coût sous-accumulée entre les points de début et de fin du chemin de sous-déformation ;

longueur du chemin de sous-déformation ;

coûts des instants de synchronisation candidats respectifs.

13. Dispositif selon l'une quelconque des revendications 9-12, dans lequel le traiteur de données électronique est également arrangé pour appliquer une fonction de coût pondéré pour appliquer des pondérations différentiées aux sorties de capteur inertiel triaxial, en particulier en appliquant une fonction de coût pondéré pour appliquer également des pondérations différentiées à la première dérivée d'ordre de chaque séquence.

14. Dispositif selon l'une quelconque des revendications 9-13, dans lequel le traiteur de données électronique est également arrangé pour calculer des instants de synchronisation à partir de la séquence modèle en exécutant les étapes suivantes :

obtenir une matrice d'auto-similarité constituée de la distance Euclidienne multidimensionnelle entre chaque paire d'instants pour la séquence capturée et le reste de la séquence ;
pour chaque ligne de la matrice d'auto-similarité, informatiser un histogramme;
obtenir une donnée d'auto-similarité en calculant la somme cumulative d'un pourcentage initial prédéterminé d'un nombre total de bacs par ligne, en particulier en calculant la somme cumulative des 20% initiaux du nombre total de bacs par ligne;
trouver le minimum local de la donnée d'auto-similarité obtenue ;
sélectionner les instants de synchronisation à partir du minimum local trouvé.

15. Moyen de stockage non-transitoire qui inclut des instructions de programme pour mettre en place un procédé pour évaluer le mouvement physique d'une tâche exécutée par un opérateur durant l'exécution d'un cycle de travail sur une station de fabrication industrielle, les instructions de programme incluant des instructions pouvant être exécutées par un traiteur de données pour exécuter le procédé de l'une quelconque des revendications 1-8.

Fig. 1

Fig. 2

**Fig. 3**

**Fig. 4**

Fig. 5

Fig. 6 (a)

Fig. 6 (b)

Fig. 7

Fig. 8

Fig. 9

Fig. 10

**EP 3 758 023 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110270135 A1
- US 20060259472 A1
- WO 201076313 A
- US 2007250286 A1
- US 2012323521 A1